# EUROPEAN PATENT APPLICATION

(11) **EP 1 935 410 A1**
(43) Date of publication of application: **25.06.2008**
(21) Application number: 06026644.2
(22) Date of filing: 22.12.2006
(51) Int. Cl.: A61K 9/16, A23L 1/00

(54) **Microcapsules**

(71) Applicant: BASF SE, 67056 Ludwigshafen (DE)
(72) Inventor: Hansen, Morten Mohr, 3450 Allerod (DK); Yde, Birgitte, 3520 Farum (DK)
(74) Representative: Benthin, Stig

(57) **Abstract**

The invention relates to microcapsules comprising one or more active substances embedded in a matrix material, said microcapsules further comprising alginate/cation complex in an amount not exceeding 20 % (w/w) included in the matrix material as well as a process for preparing such microcapsules.

The microcapsules of the invention may be used for the preparation of tablets and other product including an active substance.

## Description

### FIELD OF INVENTION

The present invention relates to microcapsules comprising active substances, a process for preparing such microcapsules as well as their use and products comprising the microcapsules.

### BACKGROUND OF THE INVENTION

Naturally occurring and modified polysaccharides and naturally occurring hydrocolloids such as alginate, caseinate, carrageenan, gelatine, pectins, gum acacia, starch and modified starch, find wide spread use as matrix materials for use in the microencapsulation of sensitive or labile active substances such as vitamins, aroma and flavour substances in food, food supplements, pharmaceuticals and agricultural products, in order to protect them from exposure to oxygen, moisture and irradiation as well as physical influences, such as pressure, and thus to avoid chemical and/or physical degradation of said active substances and to improve their storage stability.

Microencapsulated products have traditionally included gelatine, which is made from the boiled bones, skins and tendons of animals. Recently, microencapsulated products based on the use of matrix materials of plant origin have been preferred for use in some products such as vegetarian foods, kosher foods and halal foods. They may also be advantageous to use as matrix materials instead of matrix materials of animal origin due to legislative restrictions.

However, the alternative matrix materials of plant origin do not have the same ability to form the intended structure in the microcapsule as gelatine. Accordingly, it is more difficult to produce tablets, extrudates and other solid masses containing the active substances, which are sufficiently hard and can hold the active substance without degradation or loss of activity during storage.

WO 03/018186 describes a process for the preparation of a microencapsulated composition containing lipophilic compounds as well as a microencapsulated composition comprising a lipophilic compound enveloped by a surfactant which is encapsulated in an alginate matrix and further coated with an outer coating.

The object of the present invention is to provide improved microcapsules comprising one or more active substances embedded in the matrix material as well as a new process for preparation thereof.

It is a further object of the invention to provide improved microcapsules having improved hardness, storage stability and mechanical strength and improved performance during the further processing into tablets, extrudates and the like.

### SUMMARY OF THE INVENTION

The present invention relates to a microcapsule comprising one or more active substances embedded in a matrix material, said microcapsule further comprising alginate/cation complex in an amount not exceeding 20 % (w/w) included in the matrix material.

The invention further relates to a process of preparing the microcapsule, which process comprises the steps of
- providing a solution or dispersion of said matrix material,
- adding to said solution or dispersion at least one active substance,
- treating the mixture thus obtained to prepare a solution or dispersion of said at least one active substance and said matrix material,
- finely dividing and drying the solution or dispersion thus obtained to prepare a mass of particles each containing the active substance embedded in a matrix,
- adding alginate in any step of the process,
- adding cations in any step of the process.

It has surprisingly been found that by including an amount of alginate/cation complex in the matrix material, a capability of forming a gelatine-like, pellet structure is established. Hereby the microcapsule becomes more mechanically and chemically stable and becomes superior to microcapsules not including the complex in the matrix material for further processing into tablets and extrudates. The microcapsule and the resulting tablets have improved hardness, storage stability and mechanical strength. Furthermore, since the amount of alginate contained in the microcapsules is relatively low, the uptake of water during tablet production and storage is minimised. Hence, oxygen-sensitive active substances are more stable in the microcapsules.

Microcapsules having a relatively low amount of a cation/alginate complex included in the matrix material have not been described before. Alginate is a compound which is known to form structures and gel with most divalent cations, but also known to be hygroscopic. As the active substances in the microcapsule can be susceptible to oxidation, its stability is compromised by the uptake of water.

The microcapsule of the present invention has the advantage that a tablet formulation made there from may contain both large and small amounts of the microcapsule dependent on the intended use.

The term "microcapsules" as used herein means particles each comprising a matrix material having embedded therein a plurality of solid or liquid micro particles or solute molecules. Microcapsules usually have a mean diameter of about 5 mm or smaller, e.g. between 1 mm and 0.05 mm, such as between 0.6 and 0.1 mm. They can also have a diameter e.g. between 2 mm and 0.01 mm, such as between 1.5 mm and 0.2 mm.

The term "dispersion" as used herein covers both an emulsion meaning a mixture comprising liquid particles (e.g. oil droplets) dispersed in an liquid medium, e.g. water, or a suspension meaning solid particles dispersed in an liquid medium, e.g. water.

The amount of aliginate/cation complex, not exceeding 20 % (w/w) included in the matrix material, is calculated on basis of the dry matter in the matrix.

The term "alginate" according to the invention means linear unbranched polymers containing β-(1→4)-linked D-mannuronic acid (M) and a-(1→4)-linked L-guluronic acid (G) residues. One appropriate alginate is Grindsted ® Alginate FD 150.

### DETAILED DESCRIPTION OF THE INVENTION

In one embodiment of the invention the microcapsule comprises the alginate/cation complex in an amount of about 0.1-15 % (w/w), e.g. 0.1-10% (w/w), such as 0.1-5% (w/w).

In another embodiment the microcapsule comprises about 0.3-4% (w/w) alginate/cation complex, e.g. about 0.5-3% (w/w) alginate/cation complex, such as about 0.5-2.5% (w/w) alginate/cation complex or about 1.0-2.0% (w/w) alginate/cation complex.

The cation is in one embodiment an alkali metal or alkaline-earth metal ion, e.g. an alkaline-earth metal ion, such as calcium or another divalent cation.

In another embodiment the microcapsule comprises a fat-soluble active substance.

The active substances comprised in the microcapsule of the invention or the microcapsule prepared according to the present invention may be any substance, e.g. any substance which during storage, transport, handling and use requires protection, e.g. from oxygen, moisture, light radiation, and physical influences, in order to avoid physical and chemical decomposition of the substance. These active substances are further defined as being active in either a chemical or biological system.

Examples of active substances suitable for use in connection with the present invention are provitamins or vitamins, e.g. vitamin A and esters thereof, E and esters thereof, e.g. E-acetate, D and K, fatty acids (both of natural origin and obtained through a fermentative process or syntheses), e.g. mono- and polyunsaturated fatty acids, which may be added in the form of fish oil containing i.a. the (n-3) fatty acids docosahexaenoic acid (DHA) and eicosapentaenoic acid (EPA), conjugated linolenic acid (CLA), phospholipids e.g. in the form of evening primrose oil and castor oil containing i.a. the (n-6) fatty acid γ-linolenic acid, arachidonic acid, lipoic acid, carotenoids, e.g. β-carotene, lutein, lycopene, β-cryptoxanthin, astaxanthin, cantaxanthin, citranaxanthin and zeaxanthin, curcumin, benzoquinones, e.g. coenzyme Q10 (ubidecarenone), phytosterols, oils and fats.

According to one embodiment the fat soluble active substance is a fat-soluble provitamin, vitamin or carotenoid or esters thereof, e.g. vitamin E, vitamin A, vitamin D and K, or a PUFA (poly unsaturated fatty acids) or Q10.

According to other embodiments of the invention the active substance comprised is a water-soluble active substance, such as vitamin B or C;
An enzyme, e.g. amylase;
A pharmaceutical, e.g. griseofulvin, ibuprofen, benzodiazepines, phenacetin, a hormone or paracetamol; or
A nutritional supplement, such as a mineral or an isoflavone.

The matrix material of the invention may be any conventional matrix material, such as naturally occurring and modified polysaccharides and naturally occurring hydrocolloids, e.g. alginate, carrageenan, gelatine, pectins and gum acacia. Starch derived from a natural source, such as potato, wheat, maize, tapioca and rice, and modified starch are other examples of suitable matrix materials.

Modified starch prepared from the unmodified starch by (partial) degradation, depolymerisation, hydrolysis, etc is a convenient matrix material. An appropriate modified starch to be used according to the invention is OSA modified starch, e.g. sodium octenyl succinate modified starch, i.e. a starch wherein a part of the hydroxyl groups are esterified by octenyl succinyl acid, providing a surface active property of the starch.

The aqueous solution of the matrix material can optionally further contain excipients, such as hydrocolloids, dissolved carbohydrates, e.g. sorbitol and sucrose, and/or an antioxidant or oil being or containing an antioxidant.

The matrix material may further contain conventional additives such as antioxidants, e.g. t-butylhydroxytoluene (BHT), t-butylhydroxyanisole (BHA), ascorbic acid, ascorbyl palmitate, sodium ascorbate, citric acid, sodium citrate, EDTA or its salts, tocopherols, TBHQ, ethoxyquine, propyl gallate, and extracts from herbs, i.a. rosemary or oregano extract; anti-caking agents, e.g. tri-calcium phosphate and silicates, i.a. silicon dioxide and sodium aluminium silicate; plasticizers, e.g. carbohydrates and carbohydrate alcohols, examples of which are saccharose, glucose, fructose, lactose, invert sugar, sorbitol, mannitol, Trehalose, Tagatose, Pullulan, Raftilose (oligofructose), dextrin, maltodextrin, glycerin, and mixtures thereof, such as saccharose, Trehalose, Pullulan, dextrin and Raftilose and mixtures thereof.

The matrix material may further contain conventional additives such as emulsifiers and surfactants, e.g. ascorbyl palmitate, mono- and diglycerides of fatty acids and derivatives thereof, and lecithin.

In one embodiment the matrix material used in the process of the invention does not contain other divalent cations, in order to control the formation of the alginate/cation complex.

In another embodiment of the process of the invention the mass of particles is obtained by combining the matrix material and the active substance and treating the solution or dispersion. Thereafter the alginate is added to the solution or dispersion and then the cations.

It may be important that no part of the dry matter in the matrix material is physically lost during the process of the invention. Accordingly, the process is in one embodiment carried out in a way that such a loss is avoided or minimised, by postponing one of the steps wherein either the alginate or the cations are added until after the step of finely dividing and drying the solution or dispersion.

In one embodiment the addition of cations is performed by spray coating of the alginate containing microcapsules with a cation containing solution, e.g. in a fluid bed.

In another embodiment the addition of cations is performed by powdering alginate containing microcapsules with a cation containing powdering agent.

The dividing and drying of the solution or dispersion to produce a mass of particles can be done in any conventional way, such as spray cooling, spray drying or sheet drying and crushing, see e.g. WO 91/06292.

The present invention also relates to a product comprising the microcapsule(s) according to the present invention. According to one embodiment of the invention, this product is a food, a food supplement, a beverage, a pharmaceutical or veterinary product, a feed or feed supplement, a personal care product or a household product.

The products prepared according to the method of the invention are as well suitable for a wide variety of applications, such as the ones described above.

Finally, the invention relates to the use of microcapsules of the invention for the manufacture of tablets or other solid masses containing an active substance.

The process of the invention may be carried out in accordance with the following general recipe or as shown in the examples:

The water soluble ingredients, including the matrix material, are added to hot water and dissolved under continuous agitation. The oil soluble ingredients, if any, are mixed and then added to the water phase together with the active substance, and the mixture is treated to prepare a solution or dispersion. Alginate is added to the solution or dispersion, and the solution or dispersion is diluted, if necessary, to an appropriate viscosity before the solution or dispersion is finely divided and dried by a conventional method. Thereafter, the cations are added, e.g. as an aqueous solution of an appropriate salt.

The invention will now be described in further detail with reference to the following examples.

### EXAMPLES

### Preparations of microcapsules

### Example 1 (0.5% alginate)

650 ml distilled water was heated to 65°C, where after 431 g sucrose and 407 g OSA-starch (Capsul® National Starch) was added under continuous agitation. When the sucrose and Capsul® had dissolved, 1220 g vitamin E acetate oil was added under vigorous stirring. The mixture was homogenised/emulsified for about 35 minutes at 5000 rpm. After emulsification 261 g of a 4% alginate solution in distilled water was added to the emulsion. The emulsion was diluted with distilled water to an appropriate sprayable viscosity.

Subsequently the emulsion was atomised in a spray drying tower into which cornstarch was simultaneously injected. After drying the particles were spray coated with a 5 % CaCl₂ solution in water in a fluid bed.

### Example 2 (1.5% alqinate):

650 ml distilled water was heated up to 65°C, where after 431 g sucrose and 407 g OSA-starch (Capsul® National Starch) was added under continuous agitation. When the sucrose and Capsul® had dissolved, 1220 g vitamin E acetate oil was added under vigorous stirring. The mixture was homogenised/emulsified for about 35 minutes at 5000 rpm. After emulsion 775 g 4% alginate in distilled water was added to the emulsion. The emulsion was diluted with distilled water to an appropriate sprayable viscosity. Subsequently the emulsion was atomised in a spray drying tower into which cornstarch was simultaneously injected. After drying the particles were spray coated with a 5 % CaCl₂ solution in water in a fluid bed.

### Example 3 (3% alginate):

650 ml distilled water was heated up to 65°C, where after 431 g sucrose and 407 g OSA-starch (Capsul® National Starch) was added under continuous agitation. When the sucrose and Capsul® had dissolved, 1220 g vitamin E acetate oil was added under vigorous stirring. The mixture was homogenised/emulsified for about 35 minutes at 5000 rpm. After emulsion 1550 g 4% alginate in distilled water was added to the emulsion. The emulsion was diluted with distilled water to an appropriate sprayable viscosity.

Subsequently the emulsion was atomised in a spray drying tower into which cornstarch was simultaneously injected. After drying the particles were spray coated with a 5 % CaCl₂ solution in water in a fluid bed.

### Example 4

A tablet containing vitamin E acetate was produced by pressing microcapsules produced according to any one of the examples 1-3 in an amount of about 78 % microcapsules together with about 20 % microcrystalline cellulose (Avicel®) and about 2% sodium aluminium silicate (Zeolex 7A®).

### Example 5: Test of product properties

The effect of the alginate/calcium complex on the properties of the final products (tablets) was verified by a number of tests conducted on products according to the invention and similar product not including an alginate/cation complex. Tablets prepared according to example 4 were subjected to a crushing test on tablet hardness.

The hardness was measured using a Schleuninger Hardness tester 8M, and the tablets were produced in a DIAF TM120 where visual inspection of the equipment after forming the tablet and the tablets themselves indicates the quality of the material.

The results are shown in Table 1 below.

The column "products before addition of Ca" are tablets prepared from microcapsules of examples 1-3 before treatment with CaCl₂. The column "products after addition of Ca" are tablets prepared according to example 4 from microcapsules of examples 1-3 after treating with CaCl₂ in a fluid bed.

**Table 1**

| | Products before addition of Ca | Products after addition of Ca |
|---|---|---|
| | Hardness in N | Hardness in N |
| Example 1 (0.5% alginate) | 44 | 47 |
| Example 2 (1.5% alginate) | 45 | 57 |
| Example 3 (3.0% alginate) | 48 | 58 |

Clearly, an improvement in the hardness is shown for the products of the invention compared to the hardness of the products before addition of Ca.

## Claims

1. Microcapsule comprising one or more active substances embedded in a matrix material, said microcapsule further comprising alginate/cation complex in an amount not exceeding 20 % (w/w) included in the matrix material.

2. Microcapsule according to claim 1, wherein the alginate/cation complex is comprised in an amount of about 0.1-15 % (w/w), e.g. 0.1-10% (w/w), such as 0.1-5% (w/w).

3. Microcapsule according to claim 2, wherein the alginate/cation complex is comprised in an amount of about 0.3-4% (w/w), e.g. about 0.5-3% (w/w), such as about 0.5-2.5% (w/w) or about 1.0-2.0% (w/w).

4. Microcapsule according to claims 1 to 3, wherein the cation is an alkali metal or an alkaline-earth metal ion, e.g. an alkaline-earth metal ion, such as calcium or another divalent cation.

5. Microcapsule according to any one of claims 1 to 4, wherein the active substance is a fat-soluble active substance.

6. Microcapsule according to any of the claims 1 to 5, wherein the active substance is selected from the group consisting of provitamins or vitamins and esters thereof, fatty acids both of natural origin and obtained through a fermentative process, phospholipids, carotenoids, benzoquinones, phytosterols, oils and fats.

7. Microcapsule according to claim 6, wherein the active substance is vitamin E or E-acetate, vitamin A, D or K, a monounsaturated fatty acid or a PUFA (polyunsaturated fatty acid), β-carotene or Q10.

8. Microcapsule according to any of the claims 1 to 4, wherein the active substance a water-soluble active substance.

9. Microcapsule according to claim 8, wherein the active substance is vitamin B or C, an enzyme, e.g. amylase, a pharmaceutical, e.g. griseofulvin, ibuprofen, benzodiazepines, phenacetin, a hormone or paracetamol, or a nutritional supplement, e.g. a mineral or an isoflavone.

10. Microcapsule according to any one of claims 1 to 9, wherein the matrix material contains hydrocolloids, antioxidants or carbohydrates.

11. Microcapsule according to any of the claims 1 to 10, wherein the matrix material is a modified starch.

12. A process of preparing a microcapsule according to any one of claims 1 to 11, which process comprises the steps of
- providing a solution or dispersion of said matrix material,
- adding to said solution or dispersion at least one active substance,
- treating the mixture thus obtained to prepare a solution or dispersion of said at least one active substance and said matrix material,
- finely dividing and drying the mixture thus obtained to prepare a mass of particles each containing the active substance embedded in a matrix,
- adding alginate in any step of the process,
- adding cations in any step of the process.

13. Process according to claim 12, wherein the cations are added after the addition of the alginate.

14. Process according to claim 12, wherein either the alginate or the cations are added after the step of finely dividing and drying the solution or dispersion.

15. Process according to any of the claims 13 or 14, wherein the addition of cations is performed by spray coating of the alginate containing microcapsules with a cation containing solution.

16. Process according to any of the claims 13 or 14, wherein the addition of cations is performed by powdering of the alginate containing microcapsules with a cation containing powdering agent.

17. Process according to claim 12, wherein the alginate and the cations are added before treating the mixture to prepare a solution or dispersion.

18. Process according to any one of claims 12 to 17, wherein the cation is an alkali metal or an alkaline-earth metal ion, e.g. an alkaline-earth metal ion, such as calcium or another divalent cation.

19. A product comprising microcapsules according to any of claims 1-11.

20. A product according to claim 19, **characterized in that** it is a food, a food supplement, a beverage, a pharmaceutical or veterinary product, a feed or feed supplement, a personal care product or a household product.

21. Use of the microcapsules according to any one of claims 1-11 or produced according to any one of claims 12-18, for the manufacture of tablets containing an active substance.
